# EUROPEAN PATENT SPECIFICATION

(11) **EP 0 430 434 B1**
(45) Date of publication and mention of the grant of the patent: **14.09.1994**
(21) Application number: 90311725.7
(22) Date of filing: 25.10.1990
(51) Int. Cl.: C07B 39/00

(54) **Process for the preparation of fluoroaromatic and fluoroheteroaromatic compounds**
Verfahren zur Herstellung von fluoraromatischen und heterofluoraromatischen Verbindungen
Procédé pour la préparation de composés fluoroaromatiques et hétérofluoroaromatiques

(30) Priority: 22.11.1989 GB 8926430
(43) Date of publication of application: 05.06.1991
(73) Proprietor: ZENECA LIMITED, London W1Y 6LN (GB)
(72) Inventor: Milner, David John, Whitefield, Manchester, M25 7LW (GB)
(74) Representative: Giles, David Eric

(56) References cited:
- EP-A- 0 022 942
- EP-A- 0 205 019
- EP-A- 0 258 985
- GB-A- 2 173 188
- US-A- 3 422 159
- US-A- 3 481 850
- US-A- 4 476 320

## Description

This invention relates to a process for the preparation of fluoroaromatic and fluoroheteroaromatic compounds from the corresponding aromatic and heteroaromatic amines by replacement of the amino group with a fluorine atom.

A number of general methods for the preparation of fluoroaromatic and fluoroheteroaromatic compounds are known and these include:-
(i) the Schiemann reaction where a diazonium tetrafluoro borate prepared from an aromatic amine is isolated and dried and then decomposed thermally to form the fluoroaromatic compound;
(ii) diazotisation of an aromatic amine in anhydrous hydrofluoric acid and subsequent heating to produce the corresponding fluoroaromatic compound. Whilst this method is suitable for simple aromatic amines, only moderate yields of the fluoroderivative are obtained at atmospheric pressure when the amine is substituted in the ortho position with an atom having a lone pair of electrons. Additionally, special equipment is required to handle and recover hydrofluoric acid; and
(iii) halogen exchange reactions where typically a chloro- or bromoaromatic compound is reacted with an alkali metal fluoride in a suitable aprotic solvent. This method is limited to compounds which have at least one activating group, such as nitro or cyano, attached to the aromatic ring.

The Schiemann reaction was disclosed by Balz and Schiemann (Berichte, 60, 1186 (1927)). Since that time a large number of fluoroaromatic compounds have been prepared using this reaction. According to Roe's review (Organic Reactions 5, 193 (1949)) the diazonium tetrafluoro borate must be isolated and thoroughly dried before being decomposed otherwise the presence of moisture makes the decomposition uncontrollable and markedly lowers the yield. Also according to Roe the presence of hydroxyl, carboxyl, amino, nitro and ester groups lower the yield of the desired compound. This is particularly marked where an ortho substituent is present because this increases the solubility and gives a lower yield of the isolated diazonium tetrafluoro borate.

According to US Patent No.4476320 the isolation and drying of the diazonium tetrafluoro borate is necessary before decomposition of the fluoroaromatic compound.

Surprisingly it has been found that reasonable yields of a range of fluoroaromatic and fluoroheteroaromatic compounds may be obtained by the formation and direct decomposition, in situ, of diazonium tetrafluoroborate and certain related polyfluoro salts. In many cases the yields are comparable with or better than those achieved by conventional Schiemann reactions.

According to the present invention there is provided a process for the preparation of fluoroaromatic and fluoroheteroaromatic compounds by reacting the corresponding aromatic or heteroaromatic amines with a nitrosyl polyfluoro salt in an inert liquid and decomposing the derived aryl or heteroaryl diazonium polyfluoro salt in situ.

Suitable aromatic and heteroaromatic amines are of the Formula (1):

A - (NH₂)ₙ (1)

wherein:
A is a substituted or unsubstituted aromatic or heteroaromatic radical; and
n is 1 or 2.

It is preferred that any ring in the aromatic or heteroaromatic radical carries only one amino group. Thus in the monocyclic aromatic or heteroaromatic radicals represented by A it is preferred that n is 1. Where A is an aromatic or heteroaromatic radical with more than one aromatic or hetaroaromatic ring each ring may carry an amino group.

The radical represented by A is preferably aryl, especially phenyl or naphthyl; heteroaryl especially pyridyl, quinolinyl, thienyl, furanyl; or biaryl, especially biphenyl and diphenylmethyl.

The radical A may be unsubstituted or substituted by any one or more of the following groups: -H; C₁₋₆-alkyl; C₁₋₆-alkoxy; aryloxy; C₁₋₄-alkyl NH₂; -OH; -COOH; -CHO; -COOC₁₋₆-alkyl; -F; -Cl; -Br; -I; -CF₃; -NO₂; -CN; -SO₃H; -C₁₋₄-alkylarylNH₂; -C₁₋₄-alkylCOOH; -C₁₋₄-alkylCOOC₁₋₄alkyl; -NHCOC₁₋₄-alkyl; -CONR¹R², wherein R¹ and R² are each independently selected from -H and C₁₋₄-alkyl; or -COX wherein X is -CH₃, phenyl, aminophenyl or fluorophenyl.

It is preferred that when A is phenyl, it is substituted by one or more of the following groups: -H; C₁₋₄-alkyl, especially methyl or ethyl; aryloxy; C₁₋₄-alkoxy, especially methoxy or ethoxy; -OH; -COOH; -CHO; -COOCH₃; -F; -Cl; -CF₃; -NO₂; -CH₂PhNH₂; -NHCOCH₃; -COPhNH₂ or -CN.

It is preferred that when A is pyridyl it is substituted by one or more of the following groups: -H; -CH₃; -COOH; -F; -Cl; or -CF₃.

Suitable nitrosyl polyfluoro salts are of the Formula (2):

NOZFₓ (2)

wherein:
Z is boron, antimony, phosphorus, silicon or arsenic; and
x is from 4 to 6.

When n = 2 both amino groups will react with the nitrosyl polyfluoro salt to form the aryl or heteroaryl bis(diazonium polyfluoro salt) which decomposes to the corresponding difluoro aromatic or heteroaromatic compound.

Examples of suitable aromatic amines for use in this process includes
aniline;
2-, 3- and 4-aminophenols;
2-, 3- and 4-aminobenzoic acids;
2-, 3- and 4-aminonitrobenzenes;
2-, 3- and 4-aminobenzonitriles;
alkyl 2-, 3- and 4-aminobenzoates;
2-, 3- and 4-aminobenzaldehydes;
2-, 3- and 4-chloroanilines;
2-, 3- and 4-bromoanilines;
2-, 3- and 4-aminopyridines;
4-aminobenzophenone;
4,4'-diaminobenzophenone;
4,4'-diaminodiphenylmethane;
4-aminoacetophenone;
4-amino-2-chloroacetophenone;
2-, 3- and 4-aminotoluenes;
2-amino-5-methylnaphthyridine;
4-methyl-7-aminocoumarin;
1- and 2-aminoanthraquinone;
1,6-diaminoanthraquinone;
2-aminofluorenone;
4-aminobenzothiadiazole.

This process is especially suitable for the preparation in good yield of fluoroaromatic and fluoroheteroaromatic compounds from the corresponding amino or aromatic compounds and more especially those with ortho substituents such as 2-fluorophenol, 2-fluorobenzoic acid, 2-fluoronitrobenzene, 2-fluorobenzonitrile, methyl 2-fluorobenzoate, ethyl 2-fluorobenzoate, 2-fluorobenzaldehyde, 2-chlorofluorobenzene, 2-bromofluorobenzene and 2-fluoropyridine. By good yield it is meant that the yields obtained by the present process are at least as good as the yields obtained by the conventional Schiemann process.

The reaction may be conveniently carried out by dissolving or slurrying the aromatic amine in a suitable inert liquid and adding a slurry of nitrosyl polyfluoro salt in a suitable inert liquid. The aromatic amine does not need to be completely soluble in the inert liquid and reaction occurs part of the amine is suspended in the inert liquid. Alternatively the amine solution or suspension may be added to the nitrosyl polyfluoro salt slurry. The mixture is stirred until the reaction is essentially complete. Where necessary the temperature of the reaction mixture is increased, optionally after adding a suitable high boiling inert liquid, to effect decomposition to the corresponding fluoroaromatic compound. It is generally necessary to increase the temperature by up to 200°C, preferably from 5°C to 150°C, to effect decomposition

By the term "in situ" it is meant that the derived diazonium, or bis(diazonium), salt is not isolated from the other reactants used in the present process, as would be done in conventional Schiemann reactions, but it does include the possibility that the reaction liquid may be replaced by a different liquid such as higher boiling liquid which would allow decomposition to occur at a higher temperature.

The nitrosyl polyfluoro salt may be added directly or may be formed in the reaction liquid, for example nitrosyl fluoroborate may be formed in the reaction liquid by reacting a suitable alkyl nitrite with boron trifluoride and hydrogen fluoride. Suitable alkyl nitrites are those with straight or branched C₁₋₆-alkyl groups such as methyl, ethyl, propyl, butyl, pentyl and hexyl nitrites, preferably ethyl, isopropyl, n-butyl, sec-butyl, tert.butyl, isobutyl, n-pentyl and iso-pentyl nitrites.

Suitable liquids which can be used in the process are aromatic hydrocarbons, such as toluene, xylene and triisopropylbenzene; haloaromatics such as chlorobenzene, dichlorobenzene and chloronaphthalene; haloaliphatics such as dichloromethane and dichloroethane; ethers such as tetrahydrofuran, diethyl ether, dimethoxyethane and diethoxyethane; and alcohols such as methanol, ethanol, n-propanol, iso-propanol, n-butanol, iso-butanol and tert.butanol. The liquids may be used either alone or in any convenient mixture which gives reasonable solubility, i.e. of at least 0.1%, for the reactants and allows sufficiently high temperatures for decomposition to be achieved. Preferred liquids are toluene, dichlorobenzene, dichloromethane, tetrahydrofuran and ethanol.

The amount of nitrosyl polyfluoro salt which is used in the process can be varied within wide limits. Preferably from 1.0 to 5.0, especially from 1.0 to 2.0 and more especially from 1.1 to 1.5 mol of nitrosyl polyfluoro salt is used per mol of aromatic amine.

The amount of liquid to nitrosyl polyfluoro salt can be varied within wide limits. Preferably 5 to 100, especially 10 to 50 and more especially 15 to 25 parts of liquid is used per part of nitrosyl polyfluoro salt.

The amount of liquid to amine can be varied within wide limits preferably 5 to 200, especially 10 to 100 and more especially 15 to 30 parts of liquid is used per part of amine.

The diazotisation reaction may be carried out at temperatures between -30°C and 20°C, preferably between -20°C and 10°C and especially between -15°C and 5°C.

The diazotisation reaction mixture may be stirred at temperatures between -20°C and 20°C for up to 24 hours before the reaction is essentially complete.

Before decomposing the diazonium polyfluoro salt further portions of any of the aforementioned liquids may be added in amounts between 5 and 200 parts, preferably 10 and 100 parts and especially 15 and 30 parts per part of amine. However it is preferred to use a high boiling liquid so that the decomposition reaction may be carried out at temperatures up to 250°C, preferably 50°C and 225°C and especially 75°C and 200°C.

The derived fluoroaromatic and fluoroheteroaromatic compounds are useful as intermediates in the manufacture of a wide range of products which includes pharmaceuticals, herbicides, pesticides, dyestuffs and plastics. Examples of such intermediate and other end products which they may be used to prepare are: difluorodiphenylmethanes, difluorodiphenylmethanols and difluorobenzophenones.

The invention is further illustrated by the following Examples in which all parts and percentages are by weight unless otherwise indicated.

### Example 1

Nitrosyl fluoroborate (1.28 parts) was stirred in suspension in 1,2-dichlorobenzene (30 parts) and cooled to 0-5°C. Aniline (0.93 parts) in 1,2-dichlorobenzene (20 parts) was added and the mixture was stirred for 30 minutes. The mixture was warmed so that the temperature reached 110°C after 1 hour. The diazonium fluoroborate started to decompose at 85°C and give fluorobenzene (75%).

### Example 2

4-Aminophenol (1.09 parts) was dissolved in tetrahydrofuran (THF, 30 parts) and the solution was cooled to -15°C. Nitrosyl fluoroborate (1.28 parts) was added over 30 minutes and the mixture was stirred for a further 30 minutes at -15°C. 1,2-Dichlorobenzene (30 parts) was added and the mixture was warmed gently. The THF was gradually distilled off and the residue was heated gradually to 165°C to give 4-fluorophenol (30%).

### Example 3

A solution of 2-aminopyridine (0.94 parts) in methylene chloride (40 parts) was added over 1 hour to nitrosyl fluoroborate (1.28 parts) in methylene chloride (10 parts) at 5°C. The reaction mixture was stirred for 30 minutes at 5°C before warming to 40°C to give 2-fluoropyridine (69%).

### Example 4

2-Aminophenol (1.09 parts) was dissolved in methylene chloride (50 parts) and added quickly to nitrosyl fluoroborate (1.28 parts) in methylene chloride (10 parts) at 5°C. The miture was stirred at 5°C for 2 hours and for a further 18 hours at 20°C before adding 1,2-dichlorobenzene (30 parts). The mixture was gradually warmed, firstly distilling out the methylene chloride and then up to 160°C. The diazonium fluoroborate started to decompose at about 90°C and gave 2-fluorophenol (58%).

### Example 5

The procedure as for Example 4 was followed, but in place of the 2-aminophenol, 1.275 parts of 2-chloroaniline were used. Decomposition started at 60°C, heating was continued up to 172°C and 2-chlorofluorobenzene (90%) was obtained.

### Example 6

The procedure as for Example 1 was followed, but in place of the aniline, 1.72 parts of 2-bromoaniline were used. Decomposition started at 105°C, heating was continued up to 160°C and 2-bromofluorobenzene (90%) was obtained.

### Example 7

A slurry of 2-aminobenzoic acid (1.37 parts) in methylene chloride (40 parts) was added quickly to a solution of nitrosyl fluoroborate (1.28 parts) in methylene chloride (20 parts) at 5°C. The mixture was stirred at 5°C for 2 hours and for a further 18 hours at 20°C before adding 1,2-dichlorobenzene (30 parts). The mixture was gradually warmed, firstly distilling out the methylene chloride and then up to 160°C. The diazonium fluoroborate started to decompose at about 120°C and gave 2-fluorobenzoic acid (98%).

### Example 8

The procedure as for Example 7 was followed, but in place of the 2-aminobenzoic acid, 1.37 parts of 4-aminobenzoic acid were used. Decomposition started at 90°C, heating was continued up to 150°C and 4-fluorobenzoic acid (100%) was obtained.

### Example 9

Nitrosyl fluoroborate (1.06 parts) was stirred in methylene chloride (10 parts) and cooled to 5°C. A suspension of 2-aminobenzaldehyde (1 part) in methylene chloride (50 parts) was added over 30 minutes and the mixture was stirred for a further 30 minutes at 5°C. 1,2-Dichlorobenzene (30 parts) was added and the mixture was warmed. The methylene chloride was distilled off and the residue was heated to 160°C. The residue was cooled to 20°C to give 2-fluorobenzaldehyde (80%).

### Example 10

A solution of nitrosyl hexafluorophosphate (1.925 parts ex Fluorochem Ltd.) in methylene chloride (20 parts) was stirred at 5°C and aniline (0.93 parts) in methylene chloride (20 parts) was added. The mixture was stirred at 5°C for 1 hour before adding 1,2-dichlorobenzene (20 parts). The mixture was gradually warmed, firstly distilling out the methylene chloride and then gradually up to 160°C. The diazonium fluoroborate started to decompose at about 110°C and gave fluorobenzene (58%).

### Examples 11 - 35

The procedure of Example 9 was followed replacing the 2-aminobenzaldehyde with equimolar amounts of the appropriate amino aromatic compound.

The details are summarised in Table 1 below.

**Table 1**

| Example | Aromatic amine | Yield of F aromatic |
|---|---|---|
| 11 | 2-MeOPh-NH₂ | 70 |
| 12 | 2-PhOPh-NH₂ | 63 |
| 13 | 2-MeCOPh-NH₂ | 56 |
| 14 | 2-EtOCOPh-NH₂ | 80 |
| 15 | 4-HOCOCH₂Ph-NH₂ | 73 |
| 16 | 4-EtOCOCH₂Ph-NH₂ | 57 |
| 17 | 2-Cl-5-HOCOPh-NH₂ | 55 |
| 18 | 4-CH₃CONHPh-NH₂ | 55 |
| 19 | 2-NO₂Ph-NH₂ | 15 |
| 20 | 3-NO₂Ph-NH₂ | 65 |
| 21 | 2-FPh-NH₂ | 25 |
| 22 | 3-FPh-NH₂ | 52 |
| 23 | 4-(4-NH₂PhCH₂)Ph-NH₂ | 65 |
| 24 | 3-NH₂-pyridine | 58 |
| 25 | 4-NH₂-pyridine | 8 |
| 26 | 2-NH₂-4-Me-pyridine | 70 |
| 27 | 2-NH₂-pyrimidine | 27 |
| 28 | 1,6-diaminoanthraquinone | 46 |
| 29 | 2,4-diamino-1,3,5-trimethylbenzene | 35 |
| 30 | 1-aminoanthraquinone | 90 |
| 31 | 2-aminoanthraquinone | 90 |
| 32 | 2-aminofluorenone | 70 |
| 33 | 4-aminobenzthiadiazole | 23 |
| 34 | 2-amino-5-methyl-1,8-naphthyridine | 24 |
| 35 | 4-methyl-7-aminocoumarin | 47 |

## Claims

1. A process for the preparation of fluoroaromatic and fluoroheteroaromatic compounds which comprises reacting the corresponding aromatic or heteroaromatic amines with a nitrosyl polyfluoro salt in an inert liquid and decomposing the derived aryl or heteroaryl diazonium polyfluoro salt in situ.

2. A process for the preparation of fluoroaromatic and fluoroheteroaromatic compounds according to Claim 1 in which the aromatic and heteroaromatic amine is of the Formula (1):
A - (NH₂)ₙ (1)
wherein:
A is a substituted or unsubstituted aromatic or heteroaromatic radical; and
n is 1 or 2.

3. A process for the preparation of fluoroaromatic and fluoroheteroaromatic compounds according to Claim 1 and Claim 2 wherein the aromatic or heteroaromatic amine has one or two ortho substituents in the aromatic ring.

4. A process for the preparation of fluoroaromatic and fluoroheteroaromatic compounds according to Claim 1 and Claim 2 wherein the aromatic amine is selected from:
aniline;
2-, 3- and 4-aminophenols;
2-, 3- and 4-aminobenzoic acids;
2-, 3- and 4-aminonitrobenzenes;
2-, 3- and 4-aminobenzonitriles;
alkyl 2-, 3- and 4-aminobenzoates;
2-, 3- and 4-aminobenzaldehydes;
2-, 3- and 4-chloroanilines;
2-, 3- and 4-bromoanilines;
2-, 3- and 4-aminopyridines;
4-aminobenzophenone;
4,4'-diaminobenzophenone;
4,4'-diaminodiphenylmethane;
4-aminoacetophenone;
4-amino-2-chloroacetophenone;
2-, 3- and 4-aminotoluenes;
2-amino-5-methylnaphthyridine;
4-methyl-7-aminocoumarin;
1- and 2-aminoanthraquinone;
1,6-diaminoanthraquinone;
2-aminofluorenone; and
4-aminobenzothiadiazole.

5. A process for the preparation of fluoroaromatic and fluoroheteroaromatic compounds according to Claim 1 in which the nitrosyl polyfluoro salt is of the Formula (2):
NOZFₓ (2)
wherein:
Z is boron, antimony, phosphorus, silicon or arsenic; and
x is from 4 to 6.

6. A process for the preparation of fluoroaromatic and fluoroheteroaromatic compounds according to Claim 1 wherein the nitrosyl polyfluoro salt is formed in situ from an alkyl nitrite, boron trifluoride and hydrogen fluoride.

7. A process for the preparation of fluoroaromatic and fluoroheteroaromatic compounds according to Claim 6 wherein the alkyl nitrite is a C₁₋₆-alkylnitrite.

8. A process for the preparation of fluoroaromatic and fluoroheteroaromatic compounds according to Claims 6 and 7 wherein the alkyl nitrite is selected from ethyl, isopropyl, isobutyl, n-butyl, sec-butyl, tert-butyl, n-pentyl and iso-pentyl nitrite.

9. A process for the preparation of fluoroaromatic and fluoroheteroaromatic compounds according to Claim 1 wherein from 1.0 to 5.0 mols of the nitrosyl polyfluoro salt is used per mol of aromatic amine.

## Patentansprüche

1. Verfahren zur Herstellung von fluoraromatischen und heterofluoraromatischen Verbindungen, bei dem die entsprechenden aromatischen oder heteroaromatischen Amine mit einem Nitrosylpolyfluor-Salz in einer inerten Flüssigkeit umgesetzt werden und das daraus erhaltene Aryl- oder Heteroaryl-Diazoniumpolyfluor-Salz in situ zersetzt wird.

2. Verfahren zur Herstellung von fluoraromatischen und heterofluoraromatischen Verbindungen nach Anspruch 1, bei dem das aromatische und heteroaromatische Amin die folgende Formel (1) hat:
A - (NH₂)ₙ (1)
in der:
A für einen substituierten oder unsubstituierten aromatischen oder heteroaromatischen Rest steht;
und
n für 1 oder 2 steht.

3. Verfahren zur Herstellung von fluoraromatischen und heterofluoraromatischen Verbindungen nach Anspruch 1 und Anspruch 2, wobei das aromatische oder heteroaromatische Amin einen oder zwei ortho-Substituenten im aromatischen Ring aufweist.

4. Verfahren zur Herstellung von fluoraromatischen und heterofluoraromatischen Verbindungen nach Anspruch 1 und Anspruch 2, wobei das aromatische Amin aus folgendem ausgewählt ist:
Anilin;
2-, 3- und 4-Aminophenole;
2-, 3- und 4-Aminobenzoesäuren;
2-, 3- und 4-Aminonitrobenzolen;
2-, 3- und 4-Aminobenzonitrilen;
2-, 3- und 4-Aminobenzoesäurealkylestern;
2-, 3- und 4-Aminobenzaldehyden;
2-, 3- und 4-Chloranilinen;
2-, 3- und 4-Bromanilinen;
2-, 3- und 4-Aminopyridinen;
4-Aminobenzophenon;
4,4'-Diaminobenzophenon;
4,4'-Diaminodiphenylmethan;
4-Aminoacetophenon;
4-Amino-2-chloracetophenon;
2-, 3- und 4-Aminotoluolen;
2-Amino-5-methylnaphthyridin;
4-Methyl-7-aminocumarin;
1- und 2-Aminoanthrachinon;
1,6-Diaminoanthrachinon;
2-Aminofluorenon; und
4-Aminobenzothiadiazol.

5. Verfahren zur Herstellung von fluoraromatischen und heterofluoraromatischen Verbindungen nach Anspruch 1, bei dem das Nitrosylpolyfluor-Salz die folgende Formel (2) hat:
NOZFₓ (2)
in der:
Z für Bor, Antimon, Phosphor, Silicium oder Arsen steht; und
x im Bereich von 4 bis 6 liegt.

6. Verfahren zur Herstellung von fluoraromatischen und heterofluoraromatischen Verbindungen nach Anspruch 1, bei dem das Nitrosylpolyfluor-Salz in situ aus einem Alkylnitrit, Bortrifluorid und Fluorwasserstoff gebildet wird.

7. Verfahren zur Herstellung von fluoraromatischen und heterofluoraromatischen Verbindungen nach Anspruch 6, bei dem es sich bei dem Alkylnitrit um ein C₁₋₆-Alkylnitrit handelt.

8. Verfahren zur Herstellung von fluoraromatischen und heterofluoraromatischen Verbindungen nach den Ansprüchen 6 und 7, bei dem das Alkylnitrit ausgewählt ist aus Ethyl-, Isopropyl-, Isobutyl-, n-Butyl-, sek-Butyl-, tert-Butyl-, n-Pentyl- und Iso-pentyl-nitrit.

9. Verfahren zur Herstellung von fluoraromatischen und heterofluoraromatischen Verbindungen nach Anspruch 1, bei dem 1,0 bis 5,0 Mol des Nitrosylpolyfluor-Salzes pro Mol aromatisches Amin verwendet werden.

## Revendications

1. Procédé de production de composés aromatiques fluorés et hétéro-aromatiques fluorés, qui comprend la réaction des amines aromatiques ou hétéro-aromatiques correspondantes avec un sel polyfluoré de nitrosyle dans un liquide inerte et la décomposition in situ du sel polyfluoré de diazonium arylique ou hétéro-arylique obtenu.

2. Procédé de production de composés aromatiques fluorés et hétéro-aromatiques fluorés suivant la revendication 1, dans lequel l'amine aromatique ou hétéro-aromatique répond à la formule (1) :
A - (NH₂)ₙ (1)
dans laquelle :
A est un radical aromatique ou hétéro-aromatique substitué ou non substitué ; et
n a la valeur 1 ou 2.

3. Procédé de production de composés aromatiques fluorés et hétéro-aromatiques fluorés suivant la revendication 1 et la revendication 2, dans lequel l'amine aromatique ou hétéro-aromatique porte un ou deux substituants en ortho sur le noyau aromatique.

4. Procédé de production de composés aromatiques fluorés et hétéro-aromatiques fluorés suivant la revendication 1 et la revendication 2, dans lequel l'amine aromatique est choisie entre :
l'aniline ;
les 2-, 3- et 4-aminophénols ;
les acides 2-, 3- et 4-aminobenzoïques ;
les 2-, 3- et 4-aminonitrobenzènes ;
les 2-, 3- et 4-aminobenzonitriles ;
les 2-, 3- et 4-aminobenzoates d'alkyle ;
les 2-, 3- et 4-aminobenzaldéhydes ;
les 2-, 3- et 4-chloranilines ;
les 2-, 3- et 4-bromanilines ;
les 2-, 3- et 4-aminopyridines ;
la 4-aminobenzophénone ;
la 4,4'-diaminobenzophénone ;
le 4,4'-diaminodiphénylméthane ;
la 4-aminoacétophénone ;
la 4-amino-2-chloracétophénone ;
les 2-, 3- et 4-aminotoluènes ;
la 2-amino-5-méthylnaphtyridine ;
la 4-méthyl-7-aminocoumarine ;
les 1- et 2-aminoanthraquinones ;
la 1,6-diaminoanthraquinone ;
la 2-aminofluorénone ; et
le 4-aminobenzothiadiazole.

5. Procédé de production de composés aromatiques fluorés et hétéro-aromatiques fluorés suivant la revendication 1, dans lequel le sel polyfluoré de nitrosyle répond à la formule (2) :
NOZFₓ (2)
dans laquelle :
Z représente le bore, l'antimoine, le phosphore, le silicium ou l'arsenic ; et
x a une valeur de 4 à 6.

6. Procédé de production de composés aromatiques fluorés et hétéro-aromatiques fluorés suivant la revendication 1, dans lequel le sel polyfluoré de nitrosyle est formé in situ à partir d'un nitrite d'alkyle, de trifluorure de bore et de fluorure d'hydrogène.

7. Procédé de production de composés aromatiques fluorés et hétéro-aromatiques fluorés suivant la revendication 6, dans lequel le nitrite d'alkyle est un nitrite d'alkyle en C₁ à C₆.

8. Procédé de production de composés aromatiques fluorés et hétéro-aromatiques fluorés suivant les revendications 6 et 7, dans lequel le nitrite d'alkyle est choisi entre les nitrites d'éthyle, d'isopropyle, d'isobutyle, de n-butyle, de sec.-butyle, de tertio-butyle, de n-pentyle et d'isopentyle.

9. Procédé de production de composés aromatiques fluorés et hétéro-aromatiques fluorés suivant la revendication 1, dans lequel on utilise, par mole d'amine aromatique, 1,0 à 5,0 moles du sel polyfluoré de nitrosyle.
